# EUROPEAN PATENT APPLICATION

(11) **EP 1 923 070 A1**
(43) Date of publication of application: **21.05.2008**
(21) Application number: 06124161.8
(22) Date of filing: 15.11.2006
(51) Int. Cl.: A61K 39/145, C12N 7/06

(54) **Vaccine for vaccinating feline against influenza virus**

(71) Applicant: Intervet International BV, 5831 AN Boxmeer (NL)
(72) Inventor: The designation of the inventor has not yet been filed
(74) Representative: Van Gent, Marieke

(57) **Abstract**

The present invention provides a vaccine for vaccinating felines against influenza virus, comprising an inactivated influenza virus of the A (H5) type.

A vaccine according to the invention aims at protecting felines against disease caused by infection with an (avian) influenza virus, especially of the influenza A (H5) type. Furthermore the vaccine aims at preventing cats from excreting substantial amounts of such a virus into the environment in case they become infected.

A vaccine according to the invention may be based on any suitable strain of the A (H5) type influenza virus, e.g. H5N2, H5N3 or H5N6.

## Description

The present invention is concerned with a vaccine for vaccinating felines against influenza virus.

Influenza virus is a RNA virus of the family Orthomyxoviridae (the influenza viruses) capable of infecting birds and mammals. Influenza viruses have a segmented genome of eight negative sense, single strands (segments) of RNA, abbreviated as PB2, PB1, PA, HA, NP, NA, M and NS.
The HA segment encodes the hemagluttinin protein, which is an antigenic protein found in the protein coat (viral envelope) of the viral particle. The protein is involved in cellular entry of the virus. The NA segment encodes the neuraminidase, which is an antigenic glycosylated enzyme also found on the surface of the influenza viral particle. It facilitates the release of progeny virus from the cell.

There are three types of influenza viruses: A, B, and C.
Humans can be infected with influenza types A, B, and C viruses. Type C may not be a true influenza virus and usually causes only mild or asymptomatic disease. Influenza B virus usually causes a minor illness, but it does have the potential to cause more severe disease in older persons. Influenza type A viruses can infect people, birds, pigs, horses, and other animals, but wild birds are the natural hosts for these viruses. Typically, wild birds do not become sick when they are infected with avian influenza A viruses. However, domestic poultry, such as turkeys and chickens, can become very sick and die from avian influenza, and some avian influenza A viruses also can cause serious disease and death in wild birds.

Only influenza A viruses are further classified by subtype on the basis of the two main surface glycoproteins hemagglutinin (HA) and neuraminidase (NA). No different subtypes of H and N have been identified for influenza B and C. There are 16 known HA subtypes and 9 known NA subtypes for Influenza A viruses. For example, an "H5N1" virus has an HA 5 protein and an NA 1 protein.

There are two types of antigenic variation in influenza viruses, referred to as "antigenic drift" and 'antigenic shift".
Antigenic drift occurs in both influenza A and B viruses. It is part of the continuing occurrence of new influenza strains that differ form their ancestors by mutations (point mutations) in the HA and NA genes. The amount of change can be subtle or dramatic. This process allows influenza viruses to change and re-infect people repeatedly through their lifetime, and is the reason why flu vaccines need seasonal updates. The emergence of new strains may lead to the occurrence of strains that are substantially different from the previous strain and thus may result in a large flu epidemic. The most recent example of such large-scale epidemic in Hong Kong was the attack of influenza A H3N2 Sydney in the first few months of 1998.

The second type of antigenic variation "antigenic shift". A genetic shift can occur when two different influenza viruses, co-infecting the same host, exchange a whole genomic segment, thereby giving rise to a new 'reassortant" of a whole new subtype. Such a genetic shift may result in a yet unknown viral subtype -for example, from H1N1 to H2N2 in 1957 and from H2N2 to H3N2 in 1968, that will be new for the host's immune system. The occurrence of a genetic shift may give rise to an influenza pandemic. The most recent pandemic threat occurred in Hong Kong SAR. In the middle of 1997, an avian subtype (H5N1) of influenza A was found to be associated with serious disease in humans, while the H5 subtype influenza virus was previously confined to avian species and was for the first time found to infect humans. The H5N1 infection in humans was

Subtypes of influenza A that are currently circulating among people worldwide include H1N1, H1N2, and H3N2 viruses.

Currently avian influenza (H5N1) is of growing concern worldwide because of the ongoing outbreaks in Asia, Europe and Africa and the potential for pandemic spread. The virus is highly contagious and already over 200 million domestic birds have either been culled or died following infection. So far it has claimed approximately 100 human lives. Avian influenza virus has also been known to infect cats and other felidae. Indeed in 2003/2004 more than 147 tigers and two leopards in zoos in Thailand, reportedly fed on infected chicken carcasses, died or had to be euthanized for animal welfare reasons. More recently between October 2005 and February 2006 FAO field veterinarians have reported unusual high cat mortality in Iraq and Indonesia in the vicinity of H5N1 outbreaks in poultry. In addition, in February 2006 H5N1 infection was confirmed by Friedrich Loeffler Institute (the first organisation isolated feline influenza virus H5N1) in 3 dead cats on the Baltic Sea island of Ruegen (Germany) where over 100 wild birds had been found dead on the island during previous weeks.

The potential for infection of and spread between domestic cats has been shown in laboratory trials. Researchers at the Erasmus Medical Centre, Rotterdam demonstrated systemic spread of human influenza virus (H5N1) in domestic cats infected by respiratory, digestive, and cat-to-cat contact (Rimmelzwaan, et al.,Am. J. Pathol.,16891), 176-183, 2006). All cats were infected with H5N1 virus and showed clinical signs of disease such as fever, lethargy, laboured breathing, and virus was detected in throat nasal, rectal swabs and many internal organs, regardless of the original site of infection.

In light of the information above, WHO has urged more studies on bird flu infection in cats. Furthermore, countries in Europe have advised owners of pets living near H5N1 wild bird foci to keep cats indoors and dogs on a leash when taken for a walk. These recent events lead to many questions by the public and pet owners to which the veterinary profession has to respond. There is also concern regarding the exposure of pet owners and veterinarians.

To treat infected cats with available drugs would be problematic. Therefore, it is clear that a safe and efficacious vaccine is needed for feline influenza.

An experiment with fowlpox virus expressing the Avian influenza virus (AIV) H5 hemagluttinin (HA) gene derived from A/tky/Ire/83 (TROVAC AIV-H%), which was used to induce H5 HA-specific antibodies in cats has been described (Karaca et al., Clin. Diagn. Lab. Immunol., 12(11), 1340-1342, 2005). However, the vaccinated cats were not challenged with any wild type virus.

An efficacious vaccine for vaccinating cats against influenza should prevent the vaccinated cats from getting physically ill, or even die, due to a subsequent infection with the influenza virus.
But, especially in the case of domestic cats, it is also very important that the cats ability to spread live virus to their environment is significantly reduced (i.e to other, non-vaccinated cats or animals or even humans). Thus, a vaccine for vaccinating cats against influenza infection should not only prevent the cats from getting ill or die due to an influenza infection, but should preferably also significantly reduce the amount of virus a cats may excrete into their environment in amounts that would allow infection. When the cats excrete hardly any or no virus the changes that it will infect other animals or even humans are minimized.

With the present invention such a vaccine for vaccinating felines against influenza virus is provided.
The present invention provides a vaccine for vaccinating felines against influenza virus, comprising an inactivated influenza virus of the A (H5) type.

With "feline" is meant a member of the felidae family, including domestic cats, and other felines such as lions, tigers, leopard, jaguar, puma, and cheetah, and other wild cats such as the lynx, caracal, and bobcat. The most familiar feline is the domestic cat or house cat (subspecies *Felis silvestris catus*).

A vaccine according to the invention is very suitable for use in felines, such as domestic cats, that come into regular contact with other cats, but also with birds which may be infected with an avian influenza virus (such as the very pathogenic H5N1 type), and also with humans.

A vaccine according to the invention aims at protecting felines against disease caused by infection with an (avian) influenza virus, especially of the influenza A (H5) type. Furthermore the vaccine aims at preventing cats from excreting substantial amounts of such a virus into the environment in case they become infected.

There are 9 subtypes of the A (H5) type influenza virus.
Influenza vaccines for vaccinating poultry, based on inactivated A (H5) type influenza virus, are known in the art. Typically, avian influenza vaccines for vaccinating poultry are based on influenza strains such as H5N2 or H5N6 strains. An example of such a poultry vaccine is Nobilis® Influenza H5, an inactivated avian influenza type A H5N2 virus (A/Chicken/mexico/232-CPA/94) water-in-oil emulsion vaccine manufactured by Intervet International, Boxmeer, the Netherlands.
This vaccine comprises the inactivated virus and an adjuvant ("GNE" adjuvant, which is a proprietary product Intervet International, the Netherlands).
Inactivated influenza type A H5N3 based vaccine was tested in humans (Nicholson et al., Lancet, 357(9272): 1937-43, 2001). A vaccine according to the invention may be based on any suitable strain of the A (H5) type influenza virus, e.g. H5N2, H5N3 or H5N6.
Although a suitable vaccine may even be based on a H5N1 strain (optionally modified to reduce its virulence), it is preferred to base the vaccine on a subtype that is different in its Neuraminidase type from the one actually causing the infection (wild type virus). This difference in the neuraminidase type between wild type strain and vaccine strain allows for the differentiation between vaccinated and infected animals, by using differential diagnostic techniques based on the detection of the neuraminidase subtype of the virus. A vaccine that allows vaccinated animals to be discriminated from infected animals is commonly referred to as a "marker vaccine".

The strains useful in a vaccine according to the invention may be isolated wild type strains or may be strains that are created with the aid of recombinant DNA techniques such as "reverse genetics", or may be strains which are reassortants wherein segments of different existing influenza type A strains are combined (in creating reassortants, usually a segment of a wild type strain is combined with the backbone of a safe vaccine strain referred to as the "donor strain"). For each subtype different strains may exist which can be used in a vaccine according to the invention, or segments of different strains may be combined or modified. Good results were obtained with a vaccine according to the invention, based on an influenza A strain of the subtype H5N6 (strain A/duck/postdam/2243/84).

A vaccine according to the invention preferably contains a suitable adjuvant. With an adjuvant is meant a substance that increases the ability of an antigen to stimulate the immune system. Different types of adjuvants are known in the art, for example Freunds complete adjuvant (FCA), which is prepared as a water in oil emulsion and comprises a mixture of a non-metabolizable oil (mineral oil), a surfactant (Arlacel A), and mycobacteria (M. tuberculosis or M. butyricum), or Freunds incomplete adjuvant which is the same oil/surfactant mixture as FCA but does not contain any mycobacteria. Alternative adjuvants include other oil/surfactant based adjuvants in which different surfactants are combined with either a non-metabolizable mineral oil, a metabolizable oil, or a mixture of the two. They may be prepared for use as an emulsion with aqueous Ag solution. Examples of such adjuvants are Montanide ISA Adjuvants [Seppic, Paris, France]. Other adjuvants known in the art include aluminium salt adjuvants. Preferably adjuvants are used that are effective, but also well tolerated by the animal and do not give rise to severe or painful local reactions at the injection site.

Adjuvated inactivated viral vaccines for cats are known in the art.
For example, Nobivac Rabies (Intervet International BV) which contains inactivated rabies virus and is adjuvated with aluminumphosphate.

With a vaccine according to invention, good results were obtained with an adjuvant is based on an dl- alpha tocopherol (vitamin E), a substance known for its antioxidant effects on the cell membranes. Adjuvants of this type are disclosed in EP382271.
An example of such an adjuvant is Diluvac Forte (Intervet International BV).

A vaccine according to the invention can be used to vaccinate cats to prevent them from getting physically ill, or even die, from an infection with and influenza virus, more particular, a type A (H5) influenza virus such as the very pathogenic H5N1 strains.

Moreover, vaccinating felines with a vaccine according to the invention will prevent the vaccinated animal from excreting the virus into its environment. Especially in cases where the cat may get infected with a very pathogenic strain of the H5N1 virus, fear exists that the virus will be, or will become, infectious for humans as well, even when excreted by a cat. This is especially important in view of the fact that these viruses can mutate rapidly and they have a tendency to acquire genes from flu viruses that infect other animal species. If avian and human influenza viruses were to simultaneously infect a person or animal, the two viruses might swap genes. The result could be a new virus that is readily transmissible between humans and against which humans would have no natural immunity. Such an event could trigger a worldwide influenza pandemic. Thus, the vaccine will limit the spread of the virus and will thereby limit the risk that other animals or humans that may be in contact with an infected cat will get infected.

The virus that forms the basis for a vaccine according to the invention may be produced by methods known to the person skilled in the art.
State of the art influenza vaccines are at present mostly produced in fertilized chicken eggs (egg-based influenza vaccine production). The influenza virus is injected into the eggs and accumulates in the fluid surrounding the embryo. The embryo becomes infected so that the virus can multiply. After several days of incubation, the eggs are opened to harvest the virus. The harvested virus is purified, chemically inactivated and used to produce a vaccine.

An alternative way of producing flu vaccine is based on cell or tissue cultures (cell-culture based influenza vaccine production). Mammalian kidney cells are preferably used for these cell cultures. Useful cells include those known in the art such as canine kidney cells, for example MDCK cells, or monkey kidney cells such as Vero cells.

Both egg-derived- and cell-culture derived influenza virus can be used in the manufacture of a vaccine according to the invention. Based on immunization results obtained with vaccines according to the invention of both types (egg-based versus cell-culture based), cell culture derived antigen is preferred. Good results were obtained when antigen was used that was derived from a culture of MDCK cells.

A vaccine according to the invention is therefore preferably based on MDCK derived influenza virus if the H5N6 subtype (strain A/duck/postdam/2243/84), and adjuvated with a vitamin E based adjuvant such as Diluvac Forte.

The invention is further exemplified by the following examples:

### Example 1: Vaccine virus strain: subtype H5N6, strain A/duck/postdam/2243/84.

Antigen produced in two methods: 1) roller bottles were seeded with appropriate cell density of MDCK cells. After cells become confluent, the cells were infected with the virus at MOI of . The infected cells were incubated at 36°C - 38°C until CPE complete. Supertanant and cells are harvested. Normally live virus titres are between 10^{6.0}TCID₅₀/ml and 10^{8.5}TCID₅₀/ml. 2) 9-14 days old embryonated SPF chicken eggs were candled, and eggs with live and healthy embryos were disinfected and inoculated with H5N6 virus working seed via allantoic cavity (0.1ml/per egg). The inoculated eggs were incubated at 36°C ± 2°C with 50% to 60% humidity for 1 to 3 days. The allantoic fluid was harvested after being refrigerated at 2° to 8°C for a minimum of 12 hrs. Normally live virus titres are between 10^{7.0}TCID₅₀/ml and 10^{9.0}TCID₅₀/ml.

The bulk antigen was stored at 2°C to 8°C, and was inactivated by addition of formaldehyde to a final concentration of 0.1 % and by incubation at 37°C for 48 hrs. Sodium metabisulfite will be added to inactivated bulk antigen to neutralise formaldehyde residual for 30 min at room temperature. The pH was adjusted to pH7.2 by the addition of sodium bicarbonate.

### Example 2: testing of different formulations in animal trial.

The antigens derived from eggs and MDCK cells were formulated into 6 test vaccines with following adjuvants.

**Table 1: test vaccines used in animal trial.**

| Test vaccines | H5N6 antigen | Formulation | |
|---|---|---|---|
| | | Adjuvants | % antigen used |
| 1 | Egg derived | Diluvac Forte | 50 |
| 2 | Egg derived | ALPO4 | 50 |
| 3 | Egg derived | nGNE | 28 |
| 4 | MDCK derived | Diluvac Forte | 50 |
| 5 | MDCK derived | ALPO4 | 50 |
| 6 | MDCK derived | nGNE | 28 |

The finished products were aliquoted into one dose vials (1.0ml/vial) which were stored at at 2°C to 8°C.

### Animal study

Seven groups of 5 SPF cats aged between 8 and 9 weeks at the beginning of the study were used. For the first 6 groups each group were immunised subcutaneously with one dose of one of the test vaccines (test vaccines 1 to 6 above) twice on the back of the neck with a four week interval. Group 7 was kept as un-vaccinated controls.

Fur over the injection area was cut for easy examination of local reactions. All cats were clinically monitored including local reactions and rectal temperature daily. In addition, on the day of each inoculation the rectal temperature of each cat was recorded at 4 hours after each inoculation.

The 5 cats immunised with test vaccine No. 4 (MDCK cells derived antigen formulated in Diluvac Forte) (Group 4) and the 5 un-vaccinated controls (group 7) were transferred to isolation units on day 19 post the secondary vaccination. They were challenged oro-nasally with 10^{6.0}EID₅₀/dose of feline influenza H5N1 virus R606/06 hp strain on day 27 post the secondary vaccination. Oro-pharyngeal and rectal swabs were taken from each challenged cats regularly for the detection of virus.

Blood samples were collected prior to the primary vaccination to confirm the absence of antibodies to avian influenza H5N6 virus and serological conversion after vaccinations.

The study was ended on day 26 post challenge. A wide range of tissue samples collected from both vaccinates and controls were used for the detection of challenge viral genome by a real time RT-PCR.

### RESULTS

### Clinical monitoring prior to challenge

The cats in groups 1 to 3, 5 and 6 were clinically monitored between day -1 and 56 post primary immunisation, and the cats in groups 4 and 7 were clinically monitored between day -1 and 46 post primary immunisation. This was because that cats in groups 4 and 7 were transferred to isolation units for challenge on day 47 post primary vaccination.

### Local reactions post vaccinations

In general, the daily score for each cat ≤3 is not considered to be significant to practising veterinary surgeons and pet owners. The daily score for each cat ≥5, especially long lasting one (more than 14 days), is considered to be unacceptable. Individual data is listed in Tables 1 to 6.

### Group 1

Two out of five cats did not have any local reactions. Remaining 3 cats did not have score of 5 or greater in any day examined.

### Group 2

All five cats had local reactions post vaccinations. Two cats had a score of 5 for 1 or 2 days, and remaining 3 cats had a score of 5 for 20 to 25 days.

### Group 3

Three out of 5 cats in this group showed a score of 5 for 6 to 7 post vaccination. The daily scores of the remaining two were less 5, but one of them had painful reaction for 1 day.

### Group 4

Two cats did not have any local reactions. Two cats had daily score of 1 for 1 and 2 days, respectively. The remaining one had a score of 1 for 17 days.

### Group 5

All cats in this group had local reactions, and had daily scores of 5 for 6, 8, 11, 18 and 31 days, respectively.

### Group 6

Four cats in this group had a score of 5 for 1, 1, 11 and 16 days, respectively.

### Systemic reactions post vaccinations

No abnormal systemic reactions were observed in groups 1, 2 and 5.

One cat in group 3 showed increased rectal temperature of 39.7°C on the day of but prior to the primary vaccination.

One cat in group 4 had rectal temperature of 40°C on day 46 post primary vaccination.

One cat in group 6 was in poor conditions and losing bodyweight starting from day 52 post primary vaccination, and euthanized on day 55 post primary vaccination. The cause of illness was not due to the vaccine given, but remains unclear.

### Serology

In general, in avian influenza antibody HAl titre equal to and greater than 4log₂ is considered to be protective. All cats had antibodies against avian influenza H5N6 virus higher than 4.0log₂ after the first vaccination with the exception of one cat in group 1, which was seronegative. After the secondary vaccination all cats in all groups had HAI titre equal to and greater than 5.3log₂. For each adjuvant, it seems that MDCK cells derived antigen is slightly more immunogenic than egg derived antigen. Antibody titres were lower in group 1 than that in other 5 groups.

### Challenge

### Clinical signs post challenge

No abnormal clinical signs were observed in all vaccinated cats post feline influenza H5N1 virus challenge.

In contrast, all un-vaccinated controls increased their rectal temperature up to 41.5°C from day 1 post challenge. The cats became depressed and showed laboured-breathing. One cat was found dead on day 5 post challenge. Another cat was found dead on day 6 post challenge. The remaining 3 cats were euthanized on day 6 post challenge on the ground of animal welfare.

### Detection of challenge viral genome in swabs

For the real time RT-PCR, CT-value ≤30 is considered to be viral genome positive; CT-value ≥ 40 is considered to be negative; and CT-value between 31 and 39 is generally negative by virus isolation in 9-11 day old SPF chicken embryonated eggs, but occasionally positive with a very low titre. A few swab suspensions were also inoculated into allantoic cavity in 9-11 day old SPF chicken embryonated eggs for further confirmation by virus isolation and the presence or absence of challenge virus in samples was confirmed by HA.

No challenge virus was detected in the oro-pharyngeal and rectal swabs taken from 4 out of 5 vaccinates. A very small amount of virus was recovered in oro-pharyngeal and rectal swabs on 1 to 2 sampling occasions in the remaining vaccinated cat by virus isolation in eggs.

No challenge virus was detected in rectal swabs taken from the controls. However, oro-pharyngeal swabs taken from the controls contained high amount of the challenge virus.

### Detection of challenge viral genome in tissues

Spleen, brain, jejunum, liver, trachea, lung, tonsil, kidney, colon and mesenteric lymph nodes were collected from the cats died or euthanized. All these tissues from the controls contained very high amount of the challenge virus. The tissues taken from the vaccinates all had very high CT-values by the real time RT-PCR, and were more likely not harbour the challenge virus.

## Claims

1. A vaccine for vaccinating felines against infection with influenza virus, **characterised in that** said vaccine comprises an inactivated influenza virus type A (H5).

2. A vaccine according to claim 1, **characterised in that** the vaccine further comprises an adjuvant.

3. A vaccine according to claim 1 or 2, **characterised in that** the influenza virus type A (H5) is of the subtype H5N6.

4. A vaccine according to any of claims 1-3, **characterised in that** the adjuvant is a vitamin E based adjuvant.

5. A vaccine according to any of claims 1-4, **characterised in** the influenza virus is derived from a culture of MDCK cells.

6. Use of a vaccine according to any of claims 1-5 for the protection of felines against infection with an influenza virus.
